Europäisches Patentamt

European Patent Office

Office européen des brevets

(11) Numéro de publication: **0 351 303 B1**

## FASCICULE DE BREVET EUROPEEN

(45) Date de publication de fascicule du brevet: **16.03.94** (51) Int. Cl.⁵: **C12P 19/04**, C08B 37/00, C23G 1/02

(21) Numéro de dépôt: **89401982.7**

(22) Date de dépôt: **11.07.89**

Le dossier contient des informations techniques présentées postérieurement au dépôt de la demande et ne figurant pas dans le présent fascicule.

(54) **Nouvel hétéropolysaccharide BM07, procédé permettant son obtention et son application dans divers types d'industries.**

(30) Priorité: **13.07.88 FR 8809529**

(43) Date de publication de la demande:
**17.01.90 Bulletin 90/03**

(45) Mention de la délivrance du brevet:
**16.03.94 Bulletin 94/11**

(84) Etats contractants désignés:
**AT BE CH DE ES FR GB GR IT LI LU NL SE**

(56) Documents cités:
**EP-A- 0 040 445**
**EP-A- 0 138 255**

(73) Titulaire: **RHONE-POULENC CHIMIE**
**25, quai Paul Doumer**
**F-92408 Courbevoie Cédex(FR)**

(72) Inventeur: **Ullmann, Gabriel**
**Chemin de Rejallant**
**F-16700 Ruffec(FR)**
Inventeur: **Jarry, Alain**
**Résidence Tour à L'Oiseau**
**23-25, Bvd. sous Blossac**
**F-86000 Poitiers(FR)**

(74) Mandataire: **Fabre, Madeleine-France et al**
**RHONE-POULENC CHIMIE**
**Service Brevets Chimie**
**25, quai Paul Doumer**
**F-92408 Courbevoie Cédex (FR)**

**Description**

La présente invention concerne un nouvel hétéropolysaccharide, un procédé permettant son obtention par fermentation par un microorganisme, ainsi que son application dans divers types d'industries.

Les hétéropolysaccharides sont des molécules de haut poids moléculaire contenant au moins deux types de monosaccharides formant un motif de base polymérisé.

L'un des hétéropolysaccharides les plus employés dans des domaines industriels aussi variés que,notamment l'agrochimie, l'agro-alimentaire, l'industrie pétrolière, les cosmétiques, etc, est la gomme Xanthane.

Cependant, malgré ses potentialités la gomme Xanthane présente de nombreuses insuffisances parmi lesquelles on peut citer un manque de stabilité à la température, aux pH acide et alcalin ainsi que dans les milieux fortement salins.

D'autres hétéropolysaccharides ont par la suite été mis sur le marché. Parmi ces derniers on peut citer l'hétéropolysaccharide développé par la société SHELL sous la marque SHELL-FLO S [R]. Après des analyses effectuées par la demanderesse, il a pu être mis en évidence que cet hétéropolysaccharide comportait des motifs dérivés du glucose, du galactose, et de sels des acides pyruvique, succinique et acétique.

Mais cet hétéropolysaccharide a montré lui aussi des insuffisances en particulier lorsqu'il est soumis à des températures élevées, de l'ordre de 80°C. Afin d'obvier aux inconvénients précités, la demanderesse a mis au point un nouvel hétéropolysaccharide qui non seulement est stable à la température, dans des solutions salines, basiques ou acides, mais qui de plus présente de fortes propriétés rhéologiques aux faibles concentrations, un pouvoir de suspension élevé, une dissolution rapide dans l'eau de ville comme dans l'eau distillée.

Un premier objet de l'invention est donc un tel hétéropolysaccharide.

Un deuxième objet de l'invention est un procédé de préparation de cet hétéropolysaccharide.

Un troisième objet de l'invention est l'application dudit hétéropolysaccharide dans divers types d'industries.

La présente invention concerne donc un nouvel hétéropolysaccharide BM07, caractérisé en ce qu'il est susceptible d'être obtenu par fermentation d'un milieu comportant au moins une source de carbone assimilable, par une souche Agrobacterium tumefaciens I-736, un de ses recombinants ou un de ses mutants.

Ladite souche Agrobacterium tumefaciens a été déposée conformément au Traité de Budapest, auprès de la Collection Nationale de Culture des Microorganismes (CNCM), le 1er Mars 1988, où elle est publiquement accessible sous le N° I736. Cette souche provient de la Collection Nationale de Bactéries Phytopathogène et est répertoriée sous le numéro CNBP 291 dans le catalogue 1974 de l'organisme curateur.

La culture pure d'Agrobacterium tumefaciens I-736 peut être effectuée en tube gélose incliné (slant) incubé à une température comprise entre 26 et 32°C, et plus généralement entre 28 et 32°C.

A ces températures et notamment sur des milieux à base de MY agar et de Bennett agar, dont les compositions sont indiquées ci-dessous, on a pu observer la formation d'un tapis mucoïde bactérien recouvrant la totalité du slant dès 20 heures.

Les milieux d'entretien suivants ont été considérés comme particulièrement avantageux pour la culture d'Agrobacterium tumefaciens I-736 :

- Milieu MY Agar (en g/l)

| | |
|---|---|
| Soya-Peptones | 5 |
| Extrait de levure | 3 |
| Extrait de malt | 3 |
| Glucose | 10 |
| Agar | 20 |

- Milieu T G Y Agar (produit par l'institut Pasteur) (en g/l)

2

| Peptones | 5 |
|---|---|
| Extrait de levures | 2,5 |
| Glucose | 1 |
| Agar | 20 |

- Milieu Bennett Agar (en g/l)

| Peptones | 1 |
|---|---|
| Extrait de viandes | 1 |
| NZ Amine A ® (Produit par la Sté Sheffield Chemical) | 2 |
| Glucose | 10 |
| Agar | 20 |

- Milieu T.S Agar (produit par la Sté Bio-Mérieux) (en g/l)

| Bio trypcase | 17 |
|---|---|
| Bio soyase | 3 |
| $K_2HPO_4$ | 2,5 |
| NaCl | 5 |
| Glucose | 2,5 |
| Agar | 20 |

La souche Agrobacterium tumefaciens I-736 peut également être cultivée en boîte de Petri, par exemple sur milieu MY agar ou TGY agar. Dans ces conditions les colonies sont visibles dès 24 à 30 heures, et présentent les caractéristiques suivantes, à 48 heures :
- taille : 2 à 3 mm de diamètre
- aspect lisse et peu bombé
- couleur brun-jaune très clair
- colonies à bord net et moins mucoïde sur boîte de Petri que sur slant.

La souche Agrobacterium tumefaciens I-736 peut utiliser les sucres suivants :
- glucose
- saccharose
- hydrolysats d'amidon

et plus difficilement l'amidon natif et le lactose.

Le glucose et le saccharose sont des sucres préférés.

Il a pu être mis en évidence que, d'une manière générale l'hétéropolysaccharide BM07 comporte des motifs dérivés du glucose, du galactose et des acides pyruvique, succinique et acétique ou des sels de ces acides, généralement selon des proportions molaires respectivement de 5-8/1-2/0,5-2/0,05-2 de préférence de 6 -7,5 / 1 - 1,5/ 0,5 -1/0,5 - 1/0,05-0,2 et plus préférentiellement encore de 7 / 1 / 0,5 - 1 / 0,5 - 1 / 0,05 - 0,1.

Lesdits acides pyruvique, succinique et acétique se présentent généralement sous forme de sels tels que des sels de sodium, de potassium, de calcium ou d'ammonium.

Les méthodes d'analyse de l'hétéropolysaccharide BM07 qui ont permis de déterminer sa formule brute telle que spécifiée ci-dessus, ont pour principe la détermination des éléments constitutifs (sucres et acides) après hydrolyse dudit hétéropolysaccharide BM07 et dosages chromatographiques par étalonnage interne ou externe.

Ainsi, le dosage des sucres a été réalisé de la manière suivante :
100 mg d'hétéropolysaccharide BM07 sont hydrolysés en tubes hermétiques par 5 ml d'acide trifluoroacétique molaire à 105°C pendant trois à six heures.

Cette opération est suivie d'une évaporation à sec et d'une reprise du résidu sec dans 5 ml de pyridine contenant 15 mg de sorbitol en tant qu'étalon interne ; puis d'une silylation sur 1 ml de solution pyridinique par 0,9 ml d'hexamethyldisilazane. La silylation est catalysée par 0,1 ml d'acide trifluoroacétique.

Le dosage des sucres est ensuite effectué par chromatographie en phase gazeuse à détection F.I.D, sur colonne capillaire en verre de 25 m de longueur et de 0,25 mm de diamètre, chargée de phase méthylsilicone présentant une épaisseur de film de 0,14 $\mu$. Le gaz vecteur utilisé est l'hydrogène, avec un débit de 2 ml/minute.

Le dosage de l'acide pyruvique est réalisé à partir d'une solution mère obtenue par hydrolyse de 80 mg d'hétéropolysaccharide BM07 au moyen de 5 ml d'acide chlorhydrique M pendant 1 heure à 105°C, puis ajout de 2 mg d'acide cétoglutarique (constituant l'étalon interne) et ajustement à 25 ml par de l'eau distillée.

Le dosage est alors effectué par Chromatographie Liquide Haute Performance (HPLC) au moyen d'une colonne chargée de silice greffée $C_{18}$ de 5 $\mu$ de diamètre, dont la longueur est de 250 mm et le diamètre de 4,6 mm. L'éluant utilisé est un mélange 50/50 en volume d'acide phosphorique 0,02 M et d'acétonitrile. Le débit est de 1,5 ml/minute.

La détection de l'acide pyruvique se fait par Ultra-violets à 375 nm.

Le dosage de l'acide succinique se fait après hydrolyse de l'hétéropolysaccharide BM07 dans les mêmes conditions que celles utilisées pour le dosage de l'acide pyruvique. Le dosage est direct, par étalonnage externe. La solution étalon d'acide succinique utilisée contient 8 mg d'acide succinique dans 25 ml d'eau.

On utilise à nouveau la technique de la CLHP sur des colonnes Aminex HPX87H® de BIORAD®. L'éluant est l'acide sulfurique 0,01 N, et le débit est de 0,8 ml/minute. La détection de l'acide succinique est réfractométrique.

Le dosage de l'acide acétique se fait après hydrolyse de 300 à 350 mg d'hétéropolysaccharide BM07 par 5 ml d'acide trifluoroacétique 4 N à 120°C pendant trois heures. Puis on ajoute 30 mg d'acide propionique en tant qu'étalon interne et on dose par Chromatographie en phase gazeuse à détection F.I.D.

Pour le dosage, on utilise une colonne en verre de 2 m de long et de 3 mm de diamètre remplie de phase FFAP à 5 % et d'acide phosphorique à 1 % absorbés sur chromosorb G® (AW DMCS) de 80 à 100 mesh. Le gaz vecteur est l'hélium avec un débit de 30 ml/minute.

L'hétéropolysaccharide BM07, provenant de différents échantillons, présente généralement les propriétés suivantes :

I. Sa viscosité intrinsèque est comprise entre 30 et 250 dl/g, et plus particulièrement comprise entre 140 et 250 dl/g et préférentiellement comprise entre 150 et 240 dl/g.

La viscosité intrinsèque ($\eta$) telle que spécifiée est déterminée par extrapolation à concentration nulle de la viscosité réduite

$$\frac{\eta - \eta_0}{\eta_0 C},$$

formule dans laquelle :

- $\eta$ est la viscosité de la solution
- $\eta_0$ est la viscosité du solvant
- C est la concentration en hétéropolysaccharide BM07,

en utilisant l'équation du Huggins:

$$\frac{\eta - \eta_0}{\eta_0 C} = [n] + k' (n)^2 . C$$

k' étant la constante de Huggins au 1er plateau Newtonien

La viscosité spécifique

$$\frac{\eta - \eta_0}{\eta_0}$$

est mesurée de la façon suivante :

On prépare une solution mère à 0,2 g/l de l'hétéropolysaccharide BM07 dans une solution aqueuse

4

0,1 M NaCl.

On prépare ensuite une gamme de solutions comportant l'hétéropolysaccharide BM07 à des concentrations comprises entre 0,03 et 0,1 g/l par dilution de la solution mère avec la solution aqueuse 0,1 M Nacl.

Les mesures sont ensuite effectuées à 23°C au moyen d'un viscosimètre LOW SHEAR.

On trace la courbe de viscosité spécifique en fonction de la concentration et l'on extrapole à concentration nulle.

2. La masse moléculaire de l'hétéropolysaccharide BM07 mesurée par diffusion de la lumière est généralement comprise entre $6.10^6$ et $10.10^6$, de préférence comprise entre $6,5.10^6$ et $9,5.10^6$

II. L'hétéropolysaccharide BM07 présente de très bonnes propriétés rhéologiques en solution dans l'eau distillée et ceci notamment aux faibles concentrations. Mais de plus ces propriétés se maintiennent fort bien quand l'hétéropolysaccharide est soumis à l'influence de conditions sévères, en particulier aux pH fortement acide et basique, en milieu fortement ionique et à la température.

Ainsi on a pu constater que :

1. Des solutions à 0,1 % en poids de l'hétéropolysaccharide BM07 dans l'eau distillée à 25°C présentent des viscosités à 24 heures supérieures à 350 mPa.s et plus particulièrement comprises entre 400 et 700 mPa.s ; les viscosités étant mesurées à un gradient de vitesse de 1 s$^{-1}$ au moyen d'un viscosimètre LOW SHEAR.

2. L'hétéropolysaccharide BM07 montre de bonnes propriétés rhéologiques en milieu salin, et notamment dans des solutions salines à base de $CaCl_2$, de $Na_2SO_4$ et de NaCl.

Plus particulièrement, on a constaté que des solutions à 0,3 % en poids dudit hétéropolysaccharide BM07 dans une solution saline (dont la composition figure ci-dessous), présentaient généralement des viscosités à 2 heures comprises entre 2000 et 3500 mPa.s, et plus particulièrement comprises entre 2500 et 3000 mPa.s, les viscosités étant mesurées avec un rhéomètre Carrimed® à un gradient de vitesse de 1 s$^{-1}$ ; la solution saline ayant la composition suivante :

| NaCl | 91,71 g |
|---|---|
| $CaCl_2,2H_2O$ | 10,41 g |
| $Mg\,Cl_2, 6H_2O$ | 10,12 g |
| $BaCl_2, 2H_2O$ | 0,113 g |
| $NaHCo_3$ | 0,195 g |
| Eau distillée qsp 1 l | |

D'autre part des solutions à 0,2 % en poids d'hétéropolysaccharide BM07 dans des solutions aqueuses à 20 % de NaCl présentent des viscosités à 24 heures comprises entre 1600 et 2400 mPa.s et de préférence comprises entre 1700 et 2100 mPa.s, les viscosités étant mesurées au moyen d'un viscosimètre LOW SHEAR à un gradient de vitesse de 1 s$^{-1}$.

3. Des solutions aqueuses à 0,2 % en poids d'hétéropolysaccharide BM07, à pH 1,7 et à 25°C, présentent une viscosité à 24 heures comprise entre 1000 et 2500 mPa.s, plus particulièrement comprise entre 1400 et 2000 mPa.s, les viscosités étant mesurées au moyen d'un viscosimètre LOW SHEAR, à un gradient de vitesse de 1 s$^{-1}$.

4. Des solutions aqueuses à 0,2 % en poids d'hétéropolysaccharide BM07, à pH 11,8 et à 25°C, présentent une viscosité à 24 heures comprise entre 1000 et 2500 mPa.s, plus particulièrement comprise entre 1400 et 2000 mPa.s, les viscosités étant mesurées au moyen d'un viscosimètre LOW SHEAR, à un gradient de vitesse de 1 s$^{-1}$.

5. Des solutions d'hétéropolysaccharide BM07 à 0,2 % en poids dans l'eau distillée, soumises à une température de 80°C durant 24 heures, présentent généralement des viscosités comprises entre 500 et 2500 mPa s et plus particulièrement comprises entre 1000 et 2000 mPa s, les viscosités étant mesurées au moyen d'un viscosimètre LOW SHEAR, à un gradient de vitesse de 1 s$^{-1}$.

6. Des solutions aqueuses d'hétéropolysaccharide BM07 à pH 7 et à 25°C, présentent une viscosité à 24 heures comprises entre 1000 et 2500 mPa.s, et plus particulièrement comprises entre 1400 et 2000 mPa.s, les viscosités étant mesurées au moyen d'un viscosimètre LOW SHEAR, à un gradient de vitesse de 1 s$^{-1}$.

On a également constaté que l'hétéropolysaccharide BM07 présente un bon pouvoir de suspension. Le pouvoir de suspension des solutions d'hétéropolysaccharide BM07 peut être apprécié au moyen du test suivant:

une éprouvette MEGAL de 100 ml est remplie dans sa totalité (volume occupé de 130 ml) par une

EP 0 351 303 B1

solution à 0,1 % en poids d'hétéropolysaccharide BM07 dans de l'eau distillée. La densité de la solution est alors voisine de 1.

Une bille de polyamide 6,6, d'un diamètre de 3 mm et d'une densité de 1,135 est posée à la surface du liquide, sans vitesse initiale. Le temps de chute de la bille est mesuré jusqu'à ce qu'elle parvienne au fond de l'éprouvette, soit après un parcours de chute de 23,5 cm. Afin d'obtenir un temps moyen, ce test est répété plusieurs fois. Le temps moyen de chute est généralement supérieur à 2000 secondes, et plus particulièrement est compris entre 3000 et 15000 secondes.

A titre comparatif, une solution à 0,1 % en poids de gomme xanthane présente, dans les mêmes conditions, un temps de chute compris entre environ 60 et 350 secondes.

La présente invention a également trait à un procédé de préparation de l'hétéropolysaccharide BM07 tel que décrit ci-dessus.

Ce procédé de préparation dudit hétéropolysaccharide consiste en la fermentation d'un milieu comportant au moins une source de carbone assimilable, par une souche Agrobacterium tumefaciens I-736 un de ses recombinants ou un de ses mutants.

Outre ladite source de carbone assimilable, le milieu de fermentation peut aussi renfermer au moins une source d'azote, de préférence une source d'azote organique et éventuellement un ou plusieurs sels minéraux.

Le milieu est innoculé de manière classique par la souche Agrobacterium tumefaciens I-736.

Lorsque le volume du milieu de fermentation est important, on peut avantageusement l'innoculer au moyen d'un milieu innoculum, ensemencé par un milieu de préculture liquide ; ce dernier étant préalablement ensemencé lui-même par une culture pure d'Agrobacterium tumefaciens I-736.

Selon le procédé de l'invention on peut utiliser en tant que milieu innoculum, tout milieu employé classiquement à cet effet, et avantageusement un milieu de nature minérale. En tant que milieu de préculture on peut citer par exemple le milieu YM bioth DIFCO Ref. 07101 et de préférence un milieu préparé à partir des composés suivants :

| - Soya - peptones | 5 g/l |
| - Extrait de malt | 3 g/l |
| - Extrait de levure | 3 g/l |
| - Glucose ou saccharose | 10 g/l |

Le pH naturel de ce milieu est de 7 à 7,2 et n'est pas ajusté.

A titre de source organique de carbone constitutive du milieu de fermentation on peut citer des sucres tels que le glucose, le saccharose, les hydrolysats d'amidon, et éventuellement le lactose ou l'amidon natif, ainsi que les mélanges de ces sucres. Le glucose et le saccharose sont des sucres préférés. La concentration en source organique de carbone dans le milieu de fermentation peut être comprise entre 1 et 100 g/l et de préférence entre 15 et 60 g/l.

A titre de source organique d'azote on peut citer la caséine et les caséinates, les hydrolysats de poisson, les farines de blé, de maïs ou de soja, les extraits de levure (levure de boulanger, levure de bière, levures lactiques...) Les dry distillers solubles, les protéines de pomme de terre, le corn steep liquor (CSL) et les solubles de CSL qui sont obtenus par dilution du CSL, suivie d'une élimination des particules solides par centrifugation, débourbage ou décantation. Le CSL, et tout spécialement les solubles de CSL, ont été jugés comme particulièrement avantageux dans le cadre de la présente invention.

La concentration en source organique azotée dans le milieu de fermentation peut être comprise entre 3 et 80 g/l et de préférence entre 5 et 50 g/l.

On peut citer à titre de sels minéraux que l'on peut éventuellement introduire dans le milieu de fermentation, les sulfates tels que les sulfates de magnésium, de manganèse, de zinc, de fer, les carbonates tels que le carbonate de calcium, les sels de calcium solubles, les phosphates tels que les phosphates de potassium, de sodium.

La concentration de chacun de ces sels minéraux dans le milieu de fermentation peut varier entre 0,01 et 5 g/l et de préférence entre 0,05 et 2 g/l.

Le milieu de fermentation peut également renfermer des oligoéléments tels que des traces de sels de cobalt et/ou de molybdène, ainsi que des vitamines et des nucléotides.

La fermentation peut être réalisée à des pressions comprises entre 1 et 4, bar à une température comprise entre 25 et 35°C, de préférence entre 28 et 32°C, dans des conditions aérobies submergées.

Le pH du milieu de fermentation peut être compris entre 5 et 9 et de préférence entre 6 et 8. Le pH peut être ajusté, selon le cas, avec une base telle que la soude ou la potasse ou avec un acide tel que

6

l'acide sulfurique, l'acide phosphorique, l'acide chlorhydrique ou l'acide nitrique. Le milieu de fermentation, placé par exemple, dans une cuve ou un récipient de fermentation, peut être avantageusement soumis à une agitation.

Cette agitation peut être exercée par exemple au moyen d'un secoueur réciproque, d'un secoueur giratoire, d'un mobile d'agitation ou d'une colonne à bulles. Le temps de fermentation est habituellement supérieur à 30 heures, mais est généralement compris entre 40 et 90 heures.

Les rendements de fermentation sont généralement supérieurs à 40 % plus particulièrement compris entre 55-75% et tout particulièrement compris entre 60 et 75 % en poids d'hétéropolysaccharide BM07 produit par rapport à la source de carbone mise en oeuvre.

L'hétéropolysaccharide BM07 peut être séparé du milieu de fermentation.

Pour ce faire le moût de fermentation renfermant l'hétéropolysaccharide BM07 peut être avantageusement chauffé à des températures comprises entre 80 et 120°C, pendant 10 à 60 minutes et de préférence entre 15 et 45 minutes.

Le moût soumis au traitement thermique ci-dessus présente avantageusement un pH compris entre 6 et 8.

Cependant ce pH peut être ajusté si nécessaire, avec selon le cas, une base ou un acide.

Ces derniers peuvent être choisis parmi les bases et les acides mentionnés ci-dessus utilisés pour l'ajustement du pH du milieu de fermentation.

La récupération de l'hétéropolysaccharide BM07 du moût de fin de fermentation peut alors être réalisée par précipitation dudit hétéropolysaccharide au moyen d'un liquide organique miscible avec l'eau et dans lequel l'hétéropolysaccharide est insoluble ou pratiquement insoluble.

A titre de liquides organiques convenables selon la présente invention on peut citer l'acétone, les alcools tels que l'éthanol, le propanol, l'isopropanol, le butanol, le tertio-butanol.

L'isopropanol est plus particulièrement préféré dans le cadre de la présente invention.

Le volume de liquide organique utilisé est généralement 2 à 3 fois celui du volume de moût à traiter.

La précipitation de l'hétéropolysaccharide BM07 par un liquide organique peut également être réalisée en présence de sels tels que les sulfates, chlorures ou phosphates de sodium, de potassium, ou de calcium.

L'hétéropolysaccharide BM07 une fois précipité, peut ensuite être séparé du liquide organique par filtration, centrifugation ou essorage.

Les fibres obtenues peuvent être déshydratées par exemple au moyen d'acétone ou d'un alcool tel que l'éthanol, le propanol, l'isopropanol ou le tertio-butanol.

Le poids d'alcool nécessaire pour effectuer cette opération de déshydratation est généralement de 1 à 10 fois celui des fibres à traiter.

Les fibres déshydratées peuvent subir de nouvelles opérations de filtration de centrifugation ou d'essorage.

Les fibres peuvent ensuite être séchées, broyées et tamisées de façon à obtenir une poudre d'hétéropolysaccharide BM07. Cette poudre est habituellement de couleur crème-beige.

Afin d'obtenir une poudre encore plus pure, il est possible de traiter soit le moût de fermentation, soit une solution aqueuse reconstituée à partir de la poudre obtenue selon le procédé décrit ci-dessus, au moyen d'une ou plusieurs enzymes.

A titre d'enzymes pouvant convenir à cet effet, on peut citer les protéases, les mutanases, les lipoprotéases, les cellulases et les chitinases.

La purification enzymatique peut être associée ou remplacée par des procédés physiques de purification tels que les divers modes de filtration, de dialyse, ou par les différentes techniques de chromatographie.

Les moûts de fermentation et les solutions aqueuses reconstituées d'hétéropolysaccharide BM07, ayant subi ou non un traitement de purification peuvent être concentrés. La concentration peut être avantageuse dans certains cas notamment dans la mesure où les coûts de transport peuvent être ainsi réduits. De plus, les solutions concentrées peuvent être plus rapidement mises en oeuvre que les poudres d'hétéropolysaccharide BM07. La concentration peut être réalisée par des techniques telles que l'évaporation, l'ultrafiltration, ou par diafiltration.

L'hétéropolysaccharide BM07 peut être appliqué avantageusement dans de nombreux domaines industriels où sont déjà utilisés d'autres polymères hydrosolubles.

Dans ces applications, l'hétéropolysaccharide BM07 est principalement employé en tant qu'agent épaississant, agent de suspension ou en tant qu'agent stabilisant de dispersions et ceci dans une large gamme de PH, en présence ou non de sels, de tensioactifs non-ioniques ou anioniques ou d'autres additifs.

Dans ces applications, on utilise généralement de 0,001 à 2 % en poids, de préférence entre 0,1 et 1 % en poids d'hétéropolysaccharide BM07 par rapport au poids de la composition qui le renferme.

Ainsi, l'hétéropolysaccharide BM07 peut être employé :

- dans l'industrie pétrolière par exemple dans les fluides de forage, en récupération assistée du pétrole, dans les compositions utilisées pour la fracturation des formations souterraines et dans celles destinées au traitement des puits,
- dans les compositions céramiques,
- dans l'industrie alimentaire, notamment en tant qu'agent de suspension ou épaississant,
- dans les peintures, les colles, les encres,
- dans les cosmétiques, en particulier dans les shampooings, les crèmes, les lotions et les pâtes dentifrices,
- dans les compositions destinées à l'agrochimie, notamment dans les "flowables" en tant qu'agent de suspension,
- dans l'industrie papetière, notamment pour le couchage du papier,
- dans les compositions lubrifiantes,
- dans les nettoyants industriels destinés au traitement des surfaces métalliques,
- en tant qu'agent stabilisant de dispersions aqueuses diverses comme les dispersions de charbon micronisé,
- dans l'industrie textile, notamment dans les compositions pâteuses destinées à l'impression des motifs.
- dans l'industrie des explosifs.
- pour la préparation des bétons et des plâtres, notamment en vue de leur coloration.
- dans les nettoyants ménagers ou industriels, notamment en tant qu'agent épaississant et en tant qu'agent stabilisant des particules abrasives.

Tout particulièrement l'hétéropolysaccharide BM07 peut être utilisé comme épaississant dans des compositions aqueuses acides contenant en solution aqueuse un acide organique ou minéral.

A titre d'acide organique, on peut citer des acides monocarboxyliques tels l'acide formique, l'acide acétique, l'acide chloroacétique, l'acide lactique, l'acide ascorbique, l'acide tannique, des acides dicarboxyliques tels l'acide fumarique, l'acide malonique, l'acide succinique, l'acide glutarique, l'acide itaconique, l'acide tartrique, des acides tricarboxyliques comme l'acide citrique.

A titre d'acide minéral, on peut citer l'acide chlorhydrique, l'acide phosphorique, l'acide nitrique ou l'acide sulfurique.

Tous ces acides peuvent être utilisés seuls ou en mélange entre eux. Les proportions relatives d'acide et d'hétéropolysaccharide peuvent varier dans une large mesure en fonction de facteurs tels que la nature propre de chacun de ces composés, la viscosité désirée et l'application spécifique choisie.

Généralement on utilise entre 1 et 40 % d'acide, entre 0,001 et 2 % d'héteropolysaccharide BM 07 et entre 55 et 98,99 % d'eau.

Des quantités d'hétéropolysaccharide BM 07 comprise entre 0,1 et 1 % sont toutefois préférées.

Ces compositions peuvent être préparées de toute manière souhaitable par mélange des différents composés dans l'eau. Il est préférable de dissoudre initialement l'hétéropolysaccharide BM 07 dans l'eau puis d'ajouter le ou les acides.

Lesdites compositions peuvent éventuellement contenir divers autres ingrédients utilisés dans les formulations acides tels que des agents tension-actifs, des colorants, des détergents, des parfums, des bactéricides et des abrasifs.

Lesdites compositions peuvent être plus spécifiquement destinées au nettoyage des surfaces, au détartrage des surfaces en porcelaine et métallique et au décapage des surfaces métalliques.

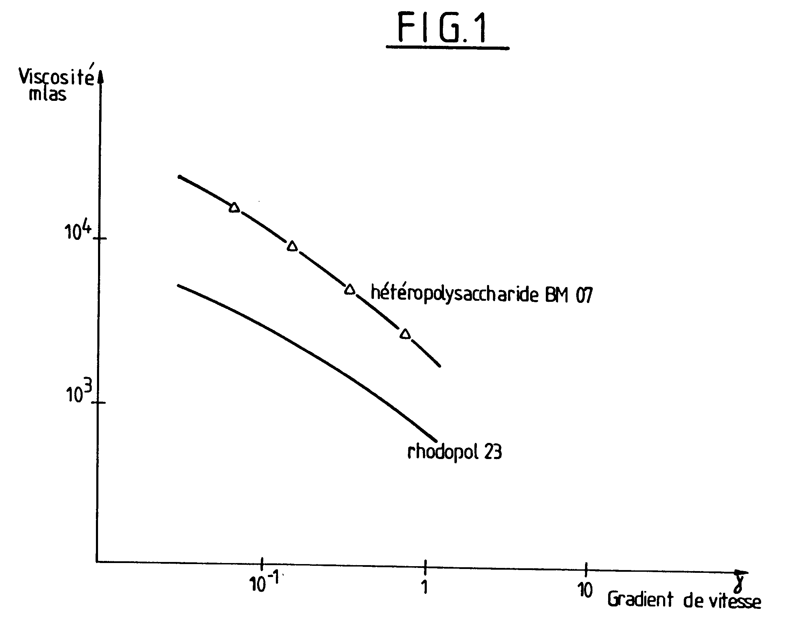

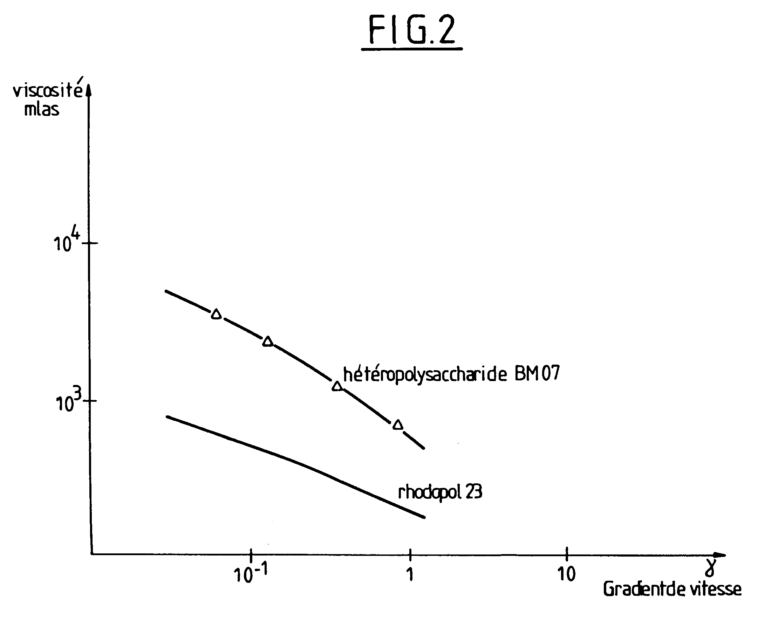

D'autres caractéristiques et avantages de l'invention apparaîtront à la lecture des exemples et des figures 1 et 2 ci-après qui représentent l'évolution de la viscosité en fonction du gradient de vitesse, de solutions dans l'eau distillée, à respectivement, 0,1 et 0,2 % en poids d'hétéropolysaccharide BM07 et de gomme xanthane.

Exemple 1 : Procédé de préparation de l'hétéropolysaccharide BM07 sur un milieu minéral de production.

On fait fermenter par une souche Agrobacterium tumefaciens I-736 un milieu renfermant (en g/l) :

| CSL (corn steep liquor) | 11 |
| $K_2 HPO_4$ | 4 |
| $MgSO_4, 7H_2O$ | 0,5 |
| Saccharose | 25 |
| Eau potable qsp | 1 l |

Ce milieu est fermenté par ladite souche à une température de 28°C, dans les conditions suivantes :
- Fermentation dans un erlenmeyer de 500 ml, contenant un volume utile de 100 ml.
  Ce milieu est soumis à une agitation de 220 tours/mn au moyen d'un secoueur giratoire sous une amplitude de 5 cm.
  Fermentation en cuve de 10 l contenant un volume utile de 6 l.
  Le milieu est soumis à une agitation de 270 tours/mn obtenue au moyen de pales à carré évidé.
  Le milieu est aéré sous un débit d'air de 500 l/h.
- Fermentation en cuve de 20 litres BIOLAFFITE® contenant un volume utile de 15 litres.
  Le milieu est soumis à une agitation de 400 tours/mn obtenue au moyen de mobiles de type RUSHTON®.
  Le milieu est aéré sous un débit d'air de 825 l/h.
Les résultats obtenus figurent dans le tableau I suivant :

TABLEAU I

| | Fin de fermentation | Rendement | Viscosité |
| --- | --- | --- | --- |
| erlenmeyer | 80 h | 72 % | 6 400 mPa.s |
| cuve de 10 l | 100 h | 67 % | 9 000 mPa.s |
| cuve de 20 l | 90 h | 66 % | 6 800 mPa.s |

Dans ce tableau I, la fin de fermentation correspond à la consommation totale ou quasi totale du saccharose et le rendement correspond au rapport en % entre le poids d'hétéropolysaccharide BM07 obtenu sur le poids de saccharose mis en oeuvre.

La viscosité est celle du moût de fin de fermentation, mesurée au moyen d'un viscosimètre Brookfield LVT® avec une aiguille cylindrique 4, à 30 tours/min.

Exemple 2 : Récupération de l'hétéropolysaccharide BM07 du moût de fermentation.

La récupération de l'hétéropolysaccharide a été effectuée à partir de 2 kg de moût obtenu par fermentation d'un milieu organique de production, contenu dans une cuve de 20 l, selon l'exemple 1.
Le moût est traité thermiquement à 90°C pendant 30 mn.
Au moût ainsi traité sont ajoutés 2300 ml d'alcool isopropylique (IPA). La précipitation est effectuée en présence de 150 g de sulfate de sodium.
Les fibres issues de la précipitation sont ensuite déshydratées 2 fois en présence de 1200 ml d'IPA.
Les fibres sont alors essorées, dilacérées et séchées dans une étuve à 85°C.
La matière sèche récoltée est broyée et tamisée.
On obtient alors une poudre d'hétéropolysaccharide BM07 de couleur crème.

Exemple 3 : Propriétés rhéologiques de l'hétéropolysaccharide BM07 dans l'eau distillée à pH7.

La viscosité et le seuil d'écoulement de solutions dans l'eau distillée à pH7, de l'hétéropolysaccharide BM07 à différentes concentrations, ont été testés.
Les essais ont été réalisés à partir d'une poudre d'hétéropolysaccharide BM07 telle qu'obtenue à l'exemple 2.
Des solutions à 0,2 % et à 0,3 %, en poids d'hétéropolysaccharide BM07 sont préparées par addition dans l'eau distillée de ladite poudre d'hétéropolysaccharide, suivie d'une agitation au moyen d'un agitateur type RAYNERI® à une vitesse de 1 000 à 1 200 tours/mn, pendant 15 mn. La mise en solution est complète au bout de ces 15 mn dans l'eau distillée.

Des solutions à 0,1 % en poids d'hétéropolysaccharide sont obtenues par simple dilution dans l'eau distillée des solutions à 0,2 % précédentes.

Les essais sont réalisés à une température de 25°C, 24 heures après la préparation des solutions.

Des essais comparatifs ont été réalisés dans les mêmes conditions avec du RHODOPOL 23 ® (gomme xanthane produite par la Société RHONE POULENC).

Les mesures des valeurs des seuils d'écoulement et des viscosités des essais 1 à 4 ont été effectuées au moyen d'un viscosimètre LOW SHEAR et au moyen d'un RHEOMAT 30 pour les mesures des essais 5 et 6.

Les résultats obtenus figurent dans le tableau II suivant :

TABLEAU II

Propriétés rhéologiques de l'hétéropolysaccharide BM07 et du Rhodopol 23 (R), en solution dans l'eau distillée à différentes concentrations

| Essai | Concentration en Hétéropolysaccharide | Seuil d'écoulement en mPa | Viscosité en mPa.s à des vitesses de cisaillement de : | | |
|---|---|---|---|---|---|
| | | | 0,03 s -1 | 0,1 s-1 | 1 s -1 |
| 1 | Hétéropolysaccharide BM07 à 0,1% | 172 | 5 020 | 2 800 | 600 |
| 2 | Rhodopol 23 (R) à 0,1% | 22 | 810 | 510 | 200 |
| 3 | Hétéropolysaccharide BM07 à 0,2% | 870 | 24 145 | 12 000 | 2 200 |
| 4 | Rhodopol 23 (R) à 0,2% | 170 | 5 200 | 3 050 | 700 |
| 5 | Hétéropolysaccharide BM07 à 0,3% | 3 155 | — | 28 000 | 3 800 |
| 6 | Rhodopol 23 (R) à 0,3% | 930 | — | 8 500 | 1 450 |

Ce tableau montre clairement que l'hétéropolysaccharide BM07 en solution dans l'eau distillée est plus pseudoplastique que la gomme xanthane placée dans les mêmes conditions.

Une solution à 0,1 % en poids d'hétéropolysaccharide présente les mêmes caractéristiques qu'une solution de gomme xanthane à 0,2 %.

Les figures 1 et 2, ci-après, illustrent l'évolution de la viscosité en fonction du gradient de vitesse, de solutions dans l'eau distillée à, respectivement, 0,1 et 0,2 % en poids d'hétéropolysaccharide BM07 et de gomme xanthane (RHODOPOL 23 ® ). Les mesures des viscosités ont été effectuées dans les mêmes conditions que celles décrites ci-dessus.

Exemple 4 : Propriétés rhéologiques de l'hétéropolysaccharide BM07 dans l'eau de ville à pH 7 à 22° HT.

Les essais ont été réalisés sur des solutions à 0,1 et 0,2 % en poids d'hétéropolysaccharide BM07 dans de l'eau de ville à pH 7, ceci dans les mêmes conditions que dans l'exemple 3.

On note cependant que la mise en solution de l'hétéropolysaccharide BM07 est encore plus facile dans l'eau distillée que dans l'eau de ville.

Les résultats des essais ainsi que ceux des essais comparatifs réalisés dans les mêmes conditions avec du RHODOPOL 23 ® , figurent dans le Tableau III suivant.

Les viscosités et les seuils d'écoulement ont été mesurés au moyen d'un viscosimètre LOW SHEAR dans les essais 7 et 9 et avec un RHEOMAT 30 pour l'essai 8.

TABLEAU III

| Essai | Concentration en Hétéropolysaccharide | Seuil d'écoulement en mPa | Viscosité en mPa.s à des vitesses de cisaillement de : | | |
|---|---|---|---|---|---|
| | | | 0,03 s- 1 | 0,1 s - 1 | 1 s -1 |
| 7 | Hétéropolysaccharide BM07 à 0,1 % | 130 | 3 750 | 1 975 | 430 |
| 8 | Rhodopol 23® à 0,1 % | 54 | – | – | 95 |
| 9 | Hétéropolysaccharide BM07 à 0,2 % | 850 | 22 940 | 10 500 | 1 750 |

EP 0 351 303 B1

Exemple 5 : Influence du pH, de la température et du cisaillement sur les propriétés rhéologiques de l'hétéropolysaccharide BM07

L'influence du pH sur les propriétés rhéologiques de l'hétéropolysaccharide BM07 a été examiné sur des solutions à 0,2 % en poids dudit hétéropolysaccharide BM07 telles que décrites dans l'exemple 3, mais où le pH est soit de 1,7 (après addition d'une quantité suffisante d'acide formique) soit de 11,8 (après addition d'une quantité suffisante de soude).

Les essais ont été réalisés à une température de 22°C, 24 heures, 7 jours et 1 mois après la préparation des solutions.

L'influence de la température a été examinée sur des solutions à 0,2 % en poids d'hétéropolysaccharide BM07 telles que décrites dans l'exemple 3.

Les mesures ont été réalisées sur de telles solutions, soumises à une température de 80°C pendant 1 heure et 24 heures.

Afin d'apprécier l'effet du cisaillement sur des solutions à 0,2 % en poids d'hétéropolysaccharide BM07 telles que décrites à l'exemple 4, on soumet ces solutions à un cisaillement par un ULTRA TURRAX JANKE-KUNKEL TP 18-20 à une vitesse maximale d'environ 20000 tours/mn pendant 5 minutes, ceci juste après la préparation des solutions. Les mesures sont effectuées 24 heures après cette opération.

Des essais comparatifs sont donnés pour des solutions à 0,2 % en RHODOPOL 23 [R] (Société Rhône-Poulenc), traitées dans les mêmes conditions.

Les mesures sont effectuées au moyen d'un viscosimètre LOW SHEAR.

Les résultats des essais figurent au Tableau IV ci-dessous :

On peut constater à la lecture de ce Tableau que la tenue des propriétés rhéologiques de l'hétéropolysaccharide BM07, soumis notamment à l'influence d'un pH acide ou basique, est très bonne, et est tout à fait comparable à la tenue dans l'eau distillée, ceci notamment sur une période de 1 mois.

TABLEAU IV

Propriétés rhéologiques de l'hétéropolysaccharide BM07 soumis à l'influence du pH, de la température et du cisaillement, et comparaison dans les mêmes conditions avec le Rhodopol 23®

| Essai | Conditions auxquelles sont soumises les solutions à 0,2% en BM07 et en Rh230 | Temps | Seuil d'écoulement en mPa | Viscosité en mPa.s à des vitesses de cisaillement de | | |
|---|---|---|---|---|---|---|
| | | | | 0,03 s$^{-1}$ | 0,1 s$^{-1}$ | 1 s$^{-1}$ |
| 10 | BM07 à pH 1,7 | 24 h<br>7 jours<br>1 mois | 720<br>760<br>830 | 19 300<br>20 485<br>22 250 | 8 750<br>9 000<br>11 000 | 1 550<br>1 600<br>1 600 |
| 11 | Rh 23 à pH 1,7 | 24 h | 30 | – | 670 | 295 |
| 12 | BM07 à pH 11,8 | 24 h<br>7 jours<br>1 mois | 830<br>900<br>1 080 | 22 530<br>24 370<br>28 940 | 10 500<br>11 000<br>13 500 | 1 800<br>1 900<br>2 100 |
| 13 | Rh 23 à pH 11,8 | | 100 | 1 850 | | 540 |
| 14 | BM07 Température de 80°C | 1 h<br>24 h | 592<br>471 | | 8 500<br>7 000 | 1 700<br>1 400 |
| 15 | Rh 23 Température de 80°C | 1 h<br>24 h | 58<br>27 | | 1 300<br>800 | 500<br>370 |
| 16 | BM07 – ULTRA TURRAX | 24 h | 711 | | 10 000 | 2 000 |
| 17 | Rh23 – ULTRA TURRAX | 24 h | 15 | | 580 | 315 |

(Mesures effectuées avec un viscosimètre LOW-SHEAR)

Dans ce Tableau : BM07 est l'hétéropolysaccharide BM07 et Rh 23 est le RHODOPOL 23®

Exemple 6 : Influence d'un milieu fortement ionique sur les propriétés rhéologiques de l'hétéropolysaccharide BM07

Les essais 18 à 23 ont été réalisés sur des solutions à 0,2 % en poids d'hétéropolysaccharide BM07 comportant 20 % de NaCl.

Ces solutions sont préparées à partir d'une solution mère à base d'eau distillée de l'hétéropolysaccharide BM07 tel qu'obtenu dans l'exemple 2 ; la solution mère est diluée par une solution NaCl jusqu'à obtention des concentrations en hétéropolysaccharide BM07 et en NaCl désirées.

Les essais ont été effectués 24 heures, 7 jours et 1 mois après la préparation des solutions, maintenues à 22°C.

Des essais comparatifs ont été réalisés dans les mêmes conditions sur des solutions à 0,2 % en RHODOPOL 23 [R] obtenues selon le même procédé. Les mesures des viscosités et des seuils d'écoulement ont été effectuées avec un viscosimètre LOW SHEAR .

Les résultats des essais figurent dans le tableau V ci-dessous.

Tableau V

Propriétés rhéologiques de l'hétéropolysaccharide BM07 et du RHODOPOL 23 (R) en solution NaCl à 20 %

| Essai | Produit | Temps | Seuil d'écoulement | Viscosité en mPa.s à des vitesses de cisaillement de : | | |
|---|---|---|---|---|---|---|
| | | | | 0,03 s -1 | 0,1 s-1 | 1 s-1 |
| 18 | hétéropolysaccharide BM07 | 24 heures | 913 | 24 535 | 11 500 | 1 800 |
| 19 | " | 7 jours | 968 | 26 100 | 12 000 | 2 000 |
| 20 | " | 1 mois | 923 | 25 150 | 12 000 | 2 000 |
| 21 | RHODOPOL 23(R) | 24 heures | 285 | 8 255 | 4 200 | 950 |
| 22 | " | 7 jours | 252 | 7 305 | 3 900 | 900 |
| 23 | " | 1 mois | 281 | 8 105 | 4 200 | 950 |

Les essais 24 à 26 et 27 à 29 ont été réalisés sur des solutions à 0,3 % en poids d'hétéropolysaccharide BM07 préparées également dans les conditions données à l'exemple 3, par addition dudit hétéropolysaccharide dans de l'eau distillée contenant 10 % en poids de 2, de $Na_2SO_4$ ou de NaCl. Les essais ont été effectués 4 heures, 7 jours et 1 mois après la préparation des solutions, maintenues dans le cas des essais 24 à 26 à 22°C et dans le cas des essais 27 à 29 à 40°C. Les mesures des viscosités ont été réalisées au moyen d'un rhéomètre BROOKFIELD L.V.T. à 60 tours/minute.

Les résultats des essais 24 à 26 et 27 à 29 figurent respectivement dans les tableaux VI et VII ci-dessous.

Tableau VI

| Propriétés rhéologiques de l'hétéropolysaccharide BM07 dans des solutions salines à 10 % et à 22°C | | | | |
|---|---|---|---|---|
| Essai | Nature du sel | Viscosité (en mPa.s) après : | | |
| | | 4 heures | 7 jours | 1 mois |
| 24 | $CaCl_2$ | 530 | 650 | 640 |
| 25 | $Na_2SO_4$ | 560 | 680 | 640 |
| 26 | NaCl | 500 | 620 | 580 |

Tableau VII

| Propriétés rhéologiques de l'hétéropolysaccharide BM07 dans des solutions salines à 10 % et à 40°C | | | | |
|---|---|---|---|---|
| Essai | Nature du sel | Viscosité (en mPa.s) après : | | |
| | | 4 heures | 7 jours | 1 mois |
| 27 | $CaCl_2$ | 550 | 610 | 630 |
| 28 | $Na_2SO_4$ | 620 | 510 | 450 |
| 29 | NaCl | 590 | 460 | - |

Exemple 7 :

Les essais 30 et 31 montrent la bonne tenue à la température des solutions d'hétéropolysaccharide BM07 comparativement à des solutions de SHELL-FLO S®.

Le SHELL-FLO S® est un hétéropolysaccharide developpé par la société SHELL et qui comporte des motifs dérivés du glucose, du galactose et des sels des acides pyruvique, acétique et succinique.

Des solutions dans l'eau distillée à 0,3 % en poids en hétéropolysaccharide BM07 sont préparées dans les conditions décrites à l'exemple 3.

De la même façon, on prépare des solutions à 0,3 % en SHELL-FLO S®.

Ces solutions sont soumises à une température de 80°C pendant 30 minutes. Après 2 heures de repos, les viscosités et les seuils d'écoulement des solutions traitées sont mesurés avec un rhéomètre Carrimed CS 50.

Les résultats figurent dans le tableau VIII suivant :

Tableau VIII

| Essai | Solution à 0,3 % en : | Seuil d'écoulement (en mPa) | Viscosité (en mPa.s) à 1 s - 1 |
|---|---|---|---|
| 30 | Hétéropolysaccharide BM07 | 1080 | 2415 |
| 31 | SHELL FLO S® | 66 | 323 |

Exemple 8 :

Utilisation de l'hétéropolysaccharide BM07 dans des compositions détartrantes ;
on réalise une composition détartrante en introduisant dans un flacon :

| - hétéropolysaccharide BM07 | 0,25 % |
|---|---|
| - eau | 87,70 % |
| - acide formique | 10 % |
| - nonylphénoléthoxylé (12 OE) | 2 % |
| - parfum et colorant | 0,05 % |

Le pH de cette composition est de 1,4.

La stabilité de cette composition est évaluée en fonction de la température et du temps de stockage, par mesure de sa viscosité au moyen d'un Rhéomat 30 dans une large gamme de gradient de vitesse (0,1s-1 à 100s-1) et d'un viscosimètre Brookfield LVT à 20 t/

Les mesures sont effectuées à 20°C et les résultats obtenus figurent dans le Tableau IX suivant :

**TABLEAU IX**

| Temps | Température en °C | Viscosité en mPa.s mesurée au Rhéomat 30 Gradient de vitesse | | | | Viscosité en mPa.s mesurée au viscosimètre Brookfield LVT à 20 t/min. |
|---|---|---|---|---|---|---|
| | | $0,1\ s^{-1}$ | $1\ s^{-1}$ | $10\ s^{-1}$ | $100\ s^{-1}$ | |
| 2 Heures | 20 | 12 500 | 2 000 | 300 | 48 | 505 |
| 8 jours | 20 | 13 000 | 1 950 | 295 | 46 | 560 |
| 1 jour | 40 | 13 500 | 2 000 | 295 | 46 | 550 |
| 8 jours | 40 | 13 000 | 1 950 | 295 | 45 | 555 |

**Revendications**

1. Hétéropolysaccharide BM07 caractérisé en ce qu'il est obtenu par fermentation d'un milieu comportant au moins une source de carbone assimilable, par une souche Agrobacterium tumefaciens I-736, un de

20

EP 0 351 303 B1

ses recombinants, ou un de ses mutants et en ce que des solutions à 0,1 % en poids dudit hétéropolysaccharide BM07 dans l'eau distillée à 25°C présentent des viscosités à 24 heures supérieures à 350 mPa.s, les viscosités étant mesurées au moyen d'un viscosimètre LOW SHEAR à un gradient de vitesse de 1 s$^{-1}$.

2. Hétéropolysaccharide BM07 selon la revendication 1 caractérisé en ce que des solutions à 0,1 % en poids dudit hétéropolysaccharide BM07 dans l'eau distillée à 25°C présentent des viscosités à 24 heures plus particulièrement comprises entre 400 et 700 mPa.s, les viscosités étant mesurées au moyen d'un viscosimètre LOW SHEAR à un gradient de vitesse de 1 s$^{-1}$.

3. Hétéropolysaccharide BM07 selon la revendication 1 ou 2 caractérisé en ce que sa viscosité intrinsèque est comprise entre 30 et 250 dl/g, et plus particulièrement comprise entre 140 et 250 dl/g et de préférence comprise entre 150 et 240 dl/g.

4. Hétéropolysaccharide BM07 selon l'une des revendications précédentes caractérisé en ce que des solutions à 0,2 % en poids d'hétéropolysaccharide BM07 dans des solutions aqueuses à 20% en poids de NaCl, présentent des viscosités à 24 heures comprises entre 1600 et 2400 mPa.s, et de préférence comprises entre 1700 et 2100 mPa.s, les viscosités étant mesurées au moyen d'un viscosimètre LOW SHEAR, à un gradient de vitesse 1 s$^{-1}$.

5. Hétéropolysaccharide BM07 selon l'une des revendications précédentes caractérisé en ce que des solutions aqueuses à 0,2 % en poids dudit hétéropolysaccharide à pH 7 et 25°C, présentent une viscosité à 24 heures comprises entre 1000 et 2500 mPa.s, et plus particulièrement comprises entre 1400 et 2000 mPa.s, les viscosités étant mesurées au moyen d'un viscosimètre LOW SHEAR, à un gradient de vitesse de 1 s$^{-1}$.

6. Hétéropolysaccharide BM07 selon l'une des revendications précédentes caractérisé en ce que des solutions aqueuses à 0,2 % en poids dudit hétéropolysaccharide, et dont le pH est de 1,7 et la température de 25°C, présentent une viscosité à 24 heures comprise entre 1000 et 2500 mPa.s, et plus particulièrement comprise entre 1400 et 2000 mPa.s, les viscosités étant mesurées au moyen d'un viscosimètre LOW SHEAR, à un gradient de vitesse s$^{-1}$.

7. Hétéropolysaccharide BM07 selon l'une des revendications précédentes caractérisé en ce que des solutions aqueuses à 0,2 % en poids dudit hétéropolysaccharide, et dont le pH est de 11,8 et la température de 25°C, présentent une viscosité à 24 heures comprise entre 1000 et 2500 mPa.s, et plus particulièrement comprise entre 1400 et 2000 mPa.s, les viscosités étant mesurées au moyen d'un viscosimètre LOW SHEAR, à un gradient de vitesse de 1 s$^{-1}$.

8. Hétéropolysaccharide BM07 selon l'une des revendications précédentes caractérisé en ce que des solutions à 0,2 % en poids dudit hétéropolysaccharide dans l'eau distillée, soumises à une température de 80°C durant 24 heures, présentent des viscosités comprises entre 500 et 2500 mPa.s, et plus particulièrement comprises entre 1000 et 2000 mPa.s, les viscosités étant mesurées au moyen d'un viscosimètre LOW SHEAR, à un gradient de vitesse de 1 s$^{-1}$.

9. Hétéropolysaccharide BM07 selon l'une des revendications précédentes caractérisé en ce qu'il présente une masse moléculaire comprise entre $6.10^6$ et $10.10^6$, de préférence comprise entre $6,5.10^6$ et $9,5.10^6$. dl/g.

10. Hétéropolysaccharide BM07 selon l'une des revendications précédentes caractérisé en ce qu'il comporte des motifs dérivés du glucose, du galactose et des acides pyruvique, succinique et acétique ou des sels de ces acides.

11. Hétéropolysaccharide BM07 selon la revendication 10 caractérisé en ce qu'il comporte des motifs dérivés du glucose, du galactose et des acides pyruvique, succinique et acétique ou des sels de ces acides selon des proportions molaires, respectivement de 5-8/1-2/0,5-2/0,5-2/0,05-2.

12. Hétéropolysaccharide BM07 selon la revendication 11 caractérisé en ce que lesdites proportions molaires sont respectivement de 6-7,5/1-1,5/0,5-1/0,5-1/0,05-0,2/.

21

**13.** Hétéropolysaccharide BM07 selon la revendication 12 caractérisé en ce que lesdites proportions molaires sont respectivement de 7/1/0,5-1/0,5-1/0,05-0,1.

**14.** Hétéropolysaccharide BM07 selon l'une des revendications précédentes caractérisé en ce que lesdits acides pyruvique, succinique et acétique se présentent sous forme de sels.

**15.** Hétéropolysaccharide BM07 selon la revendication 14 caractérisé en ce que lesdits acides se présentent sous forme de sels de sodium, de potassium, de calcium ou d'ammonium.

**16.** Procédé de préparation de l'hétéropolysaccharide BM07 selon l'une des revendications 1 à 15 caractérisé en ce qu'il met en oeuvre la fermentation d'un milieu comportant au moins une source de carbone assimilable, par une souche Agrobacterium tumefaciens I-736, un de ses recombinants, ou un de ses mutants, ladite souche de carbone assimilable étant le glucose, la saccharose ou un hydrolysat d'amidon.

**17.** Procédé selon l'une des revendications 15 et 16 caractérisé en ce que la concentration en source de carbone assimilable présente dans le milieu de fermentation est comprise entre 1 et 100 g/l et de préférence comprise entre 15 et 60 g/l.

**18.** Procédé selon l'une des revendications 15 à 17 caractérisé en ce que le milieu de fermentation comprend au moins une source d'azote organique.

**19.** Procédé selon la revendication 17 caractérisé en ce que la source d'azote organique peut être la caséine et les caséinates, les farines de blé, de maïs ou de soja, les extraits de levures, les dry distillers solubles, les protéines de pomme de terre, le corn steep liquor et les solubles de corn steep liquor.

**20.** Procédé selon la revendication 19 caractérisé en ce que la source d'azote organique est le corn steep liquor ou les solubles de corn steep liquor.

**21.** Procédé selon l'une des revendications 18 à 20 caractérisé en ce que la concentration en source d'azote organique dans le milieu de fermentation est comprise entre 3 et 80 g/l.

**22.** Procédé selon l'une des revendications 15 à 21 caractérisé en ce que le milieu de fermentation comprend au moins un sel minéral.

**23.** Procédé selon la revendication 22 caractérisé en ce que le sel minéral est un sulfate ou un carbonate.

**24.** Procédé selon l'une des revendications 21 à 23 caractérisé en ce que la concentration en sel minéral est comprise entre 0,01 et 5 g/l.

**25.** Procédé selon l'une des revendications 15 à 24 caractérisé en ce qu'il est réalisé à des pressions comprises entre 1 et 4 bar, à des températures comprises entre 25 et 35°C dans des conditions aérobies submergées.

**26.** Procédé selon l'une des revendications 15 à 25 caractérisé en ce que le pH du milieu de fermentation est compris entre 5 et 9 et de préférence entre 6 et 8.

**27.** Procédé selon l'une des revendications 15 à 26 caractérisé en ce que les rendements de fermentation sont de 40 à 75 % en poids d'hétéropolysaccharide BM07 produit par rapport à la source de carbone mise en oeuvre.

**28.** Procédé selon l'une des revendications 15 à 27 caractérisé en ce que l'hétéropolysaccharide BM07 est séparé du moût de fermentation selon les étapes suivantes:
- on chauffe le moût de fermentation entre 80 et 120°C pendant 10 à 60 minutes.
- on précipite ledit hétéropolysaccharide BM07 au moyen d'un liquide organique miscible avec l'eau.

EP 0 351 303 B1

- on sépare l'hétéropolysaccharide BM07 du liquide organique par filtration, centrifugation ou essorage.

**29.** Procédé selon la revendication 28 caractérisé en ce que ledit liquide organique est l'éthanol, le propanol, l'isopropanol, le butanol, le tertio-butanol.

**30.** Procédé selon l'une des revendications 28 et 29 caractérisé en ce qu'une fois l'hétéropolysaccharide BM07 séparé du moût de fermentation, il est déshydraté, séché, broyé et tamisé.

**31.** Application de l'hétéropolysaccharide BM07 selon l'une des revendications 1 à 15 en tant qu'agent épaississant.

**32.** Application de l'hétéropolysaccharide BM07 selon l'une des revendications 1 à 15 en tant qu'agent de suspension.

**33.** Application de l'hétéropolysaccharide BM07 selon l'une des revendications 1 à 15 dans les industries pétrolières, agrochimique, alimentaire, cosmétique, papetière, textile, des explosifs ainsi que dans les compositions céramiques et lubrifiantes, dans les peintures, les colles, les encres, les bétons, les plâtres, les nettoyants ménagers ou industriels et en tant qu'agent stabilisant de dispersions aqueuses.

**34.** Application de l'hétéropolysaccharide BM07 selon l'une des revendications 1 à 15 en tant qu'agent épaississant dans des compositions aqueuses acides contenant en solution aqueuse un acide organique ayant une constante de dissociation pk à 25°C supérieure ou égale à 2 et/ou au moins un sel d'un acide organique ou minéral de pk supérieur ou égal à 2 et d'une base forte.

**35.** Application de l'hétéropolysaccharide BM07 selon la revendication 34 caractérisé en ce que ledit acide est l'acide formique, l'acide acétique, l'acide citrique ou l'acide tannique.

**36.** Application de l'hétéropolysaccharide BM07 selon l'une des revendications 34 et 35 caractérisé en ce que lesdites compositions sont destinées au nettoyage des surfaces, au détartrage des surfaces en porcelaine et au décapage des surfaces métalliques.

**Claims**

**1.** BM07 heteropolysaccharide characterized in that it is obtained by fermentation of a medium containing at least one assimilable carbon source, by an <u>Agrobacterium tumefaciens</u> I-736 strain, one of its recombinants or one of its mutants, and in that 0.1 % by weight solutions of the said BM07 heteropolysaccharide in distilled water at 25°C have 24 hour viscosities greater than 350 mPa.s, the viscosities being measured by means of a low shear viscometer, at a speed gradient of $1 \text{ s}^{-1}$.

**2.** BM07 heteropolysaccharide according to claim 1, characterized in that 0.1 % by weight solutions of the said BM07 heteropolysaccharide in distilled water at 25°C have 24 hour viscosities more particularly between 400 and 700 mPa.s, the viscosities being measured by means of a low shear viscometer, at a speed gradient of $1 \text{ s}^{-1}$.

**3.** BM07 heteropolysaccharide according to claim 1 or 2, characterized in that its intrinsic viscosity is between 30 and 250 dl/g more particularly between 140 and 250 dl/g and preferably between 150 and 240 dl/g.

**4.** BM07 heteropolysaccharide according to one of the preceding claims, characterized in that 0.2 % by weight solutions of BM07 heteropolysaccharide in 20 % by weight aqueous solutions of NaCl have 24 hour viscosities of between 1,600 and 2,400 mPa.s, and preferably between 1,700 and 2,100 mPa.s, the viscosities being measured by means of a low shear viscometer, at a speed gradient of $1 \text{ s}^{-1}$.

**5.** BM07 heteropolysaccharide according to one of the preceding claims, characterized in that 0.2 % by weight aqueous solutions of the said heteropolysaccharide at pH 7 and 25°C, have a 24 hour viscosity of between 1,000 and 2,500 mPa.s, and more particularly between 1,400 and 2,000 mPa.s, the viscosities being measured by means of a low shear viscometer, at a speed gradient of $1 \text{ s}^{-1}$.

23

6. BM07 heteropolysaccharide according to one of the preceding claims, characterized in that 0.2 % by weight aqueous solutions of the said heteropolysaccharide, of pH 1.7 and at a temperature of 25 °C, have a 24 hour viscosity of between 1,000 and 2,500 mPa.s, and more particularly between 1,400 and 2,000 mPa.s, the viscosities being measured by means of a low shear viscometer, at a speed gradient of 1 $s^{-1}$.

7. BM07 heteropolysaccharide according to one of the preceding claims, characterized in that 0.2 % by weight aqueous solutions of the said heteropolysaccharide, of pH 11.8 and at a temperature of 25 °C, have a 24 hour viscosity of between 1,000 and 2,500 mPa.s, and more particularly between 1,400 and 2,000 mPa.s, the viscosities being measured by means of a low shear viscometer, at a speed gradient of 1 $s^{-1}$.

8. BM07 heteropolysaccharide according to one of the preceding claims, characterized in that 0.2 % by weight solutions of the said heteropolysaccharide in distilled water, subjected to a temperature of 80 °C for 24 hours, have viscosities of between 500 and 2,500 mPa.s, and more particularly between 1,000 and 2,000 mPa.s, the viscosities being measured by means of a low shear viscometer, at a speed gradient of 1 $s^{-1}$.

9. BM07 heteropolysaccharide according to one of the preceding claims, characterized in that it has a molecular mass of between $6 \times 10^6$ and $10 \times 10^6$, preferably between $6.5 \times 10^6$ and $9.5 \times 10^6$.

10. BM07 heteropolysaccharide according to one of the preceding claims, characterized in that it comprises units derived from glucose, from galactose and from pyruvic, succinic and acetic acids or salts of these acids.

11. BM07 heteropolysaccharide according to claim 10, characterized in that it comprises units derived from glucose, galactose and pyruvic, succinic and acetic acids or salts of these acids in the respective molar proportions of 5-8/1-2/0.5-2/0.5-2/0.05-2.

12. BM07 heteropolysaccharide according to claim 11, characterized in that the said molar proportions are respectively 6-7.5/1-1.5/0.5-1/0.5-1/0.05-0.2.

13. BM07 heteropolysaccharide according to claim 12, characterized in that the said molar proportions are respectively 7/1/0.5-1/0.5-1/0.05-0.1.

14. BM07 heteropolysaccharide according to one of the preceding claims, characterized in that the said pyruvic, succinic and acetic acids are present in the form of salts.

15. BM07 heteropolysaccharide according to claim 14, characterized in that the said acids are present in the form of sodium, potassium, calcium or ammonium salts.

16. Process for the preparation of the BM07 heteropolysaccharide according to one of claims 1 to 15, characterized in that it employs the fermentation of a medium containing at least one assimilable carbon source, by an <u>Agrobacterium tumefaciens</u> I-736 strain, one of its recombinants or one of its mutants, the said source of assimilable carbon being glucose, sucrose or a starch hydrolysate.

17. Process according to one of claims 15 and 16, characterized in that the concentration of the assimilable carbon source present in the fermentation medium is between 1 and 100 g/l, and preferably between 15 and 60 g/l.

18. Process according to one of claims 15 to 17, characterized in that the fermentation medium contains at least one source of organic nitrogen.

19. Process according to claim 17, characterized in that the source of organic nitrogen can be casein and caseinates, wheat, maize or soya flours, yeast extracts, dry distiller's solubles, potato proteins, corn steep liquor and corn steep liquor solubles.

**20.** Process according to claim 19, characterized in that the source of organic nitrogen is corn steep liquor or corn steep liquor solubles.

**21.** Process according to one of claims 18 to 20, characterized in that the concentration of a source of organic nitrogen in the fermentation medium is between 3 and 80 g/l.

**22.** Process according to one of claims 15 to 21, characterized in that the fermentation medium contains at least one inorganic salt.

**23.** Process according to claim 22, characterized in that the inorganic salt is a sulphate or a carbonate.

**24.** Process according to one of claims 21 to 23, characterized in that the concentration of inorganic salt is between 0.01 and 5 g/l.

**25.** Process according to one of claims 15 to 24, characterized in that it is carried out at pressures of between 1 and 4 bar, at temperatures of between 25 and 35°C, in submerged aerobic conditions.

**26.** Process according to one of claims 15 to 25, characterized in that the pH of the fermentation medium is between 5 and 9, and preferably between 6 and 8.

**27.** Process according to one of claims 15 to 26, characterized in that the fermentation yields are from 40 to 75 % by weight of BM07 heteropolysaccharide produced with respect to the carbon source used.

**28.** Process according to one of claims 15 to 27, characterized in that the BM07 heteropolysaccharide is separated from the fermented wort according to the following stages:
- The fermented wort is heated to between 80 and 120°C for 10 to 60 minutes.
- The said BM07 heteropolysaccharide is precipitated by means of an organic liquid which is miscible with water.
- The BM07 heteropolysaccharide is separated from the organic liquid by filtration, centrifugation or draining.

**29.** Process according to claim 28, characterized in that the said organic liquid is ethanol, propanol, isopropanol, butanol or tert-butanol.

**30.** Process according to one of claims 28 or 29, characterized in that once the BM07 heteropolysaccharide is separated from the fermented wort, it is dehydrated, dried, ground and sieved.

**31.** Application of the BM07 heteropolysaccharide according to one of claims 1 to 15 as a thickening agent.

**32.** Application of the BM07 heteropolysaccharide according to one of claims 1 to 15 as a suspension agent.

**33.** Application of the BM07 heteropolysaccharide according to one of claims 1 to 15 in the petroleum, agricultural chemistry, food, cosmetics, papermaking, textile and-explosives industries, as well as in ceramic and lubricant compositions, in paints, glues, inks, concretes, plasters and household or industrial cleaners, and as a stabilizing agent for aqueous dispersions.

**34.** Application of the BM07 heteropolysaccharide according to one of claims 1 to 15 as a thickening agent in aqueous acid compositions containing, in aqueous solution, an organic acid having a dissociation constant pK at 25°C greater than or equal to 2 and/or at least one salt of an organic or inorganic acid of pK greater than or equal to 2 and of a strong base.

**35.** Application of the BM07 heteropolysaccharide according to claim 34, characterized in that the said acid is formic acid, acetic acid, citric acid or tannic acid.

**36.** Application of the BM07 heteropolysaccharide according to one of claims 34 and 35, characterized in that the said compositions are intended for cleaning surfaces, descaling porcelain surfaces and pickling metallic surfaces.

**Patentansprüche**

1.  Heteropolysaccharid BM07,
    gekennzeichnet dadurch, daß es erhalten wird durch Fermentation eines Mediums, welches mindestens eine Quelle assimilierbaren Kohlenstoffs enthält, mit eine Stamm Agrobacterium tumefaciens I-736, einer seiner Rekombinanten oder einer seiner Mutanten und daß Lösungen mit 0,1 Gew.-% des Heteropolysaccharids BM07 in destilliertem Wasser bei 25°C Viskositäten nach 24 Stunden über 350 mPa•s aufweisen, wobei die Viskositäten mittels eines LOW SHEAR Viskosimeters mit einem Geschwindigkeitsgradienten von 1 s$^{-1}$ gemessen werden.

2.  Heteropolysaccharid BM07 gemäß Anspruch 1,
    gekennzeichnet dadurch, daß Lösungen mit 0,1 Gew.-% des Heteropolysaccharids BM07 in destilliertem Wasser bei 25°C Viskositäten nach 24 Stunden insbesondere zwischen 400 und 700 mPa•s aufweisen, wobei die Viskositäten mittels eines LOW SHEAR Viskosimeters mit einem Geschwindigkeitsgradienten von 1 s$^{-1}$ gemessen werden.

3.  Heteropolysaccharid BM07 gemäß einem der Ansprüche 1 oder 2,
    gekennzeichnet dadurch, daß seine intrinsische Viskosität zwischen 30 und 250 dl/g liegt und insbesondere zwischen 140 und 250 dl/g liegt und vorzugsweise zwischen 150 und 240 dl/g liegt.

4.  Heteropolysaccharid BM07 gemäß einem der vorangegangenen Ansprüche,
    gekennzeichnet dadurch, daß Lösungen mit 0,2 Gew.-% des Heteropolysaccharids BM07 in wässrigen Lösungen mit 20 Gew.-% NaCl Viskositäten nach 24 Stunden zwischen 1600 und 2400 mPa•s, vorzugsweise zwischen 1700 und 2100 mPa•s aufweisen, wobei die Viskositäten mittels eines LOW SHEAR Viskosimeters mit einem Geshwindigkeitsgradienten von 1 s$^{-1}$ gemessen werden.

5.  Heteropolysaccharid BM07 gemäß einem der vorangegangenen Ansprüche,
    gekennzeichnet dadurch, daß wäßrige Lösungen mit 0,2 Gew.-% des Heteropolysaccharids bei pH 7 und 25°C eine Viskosität nach 24 Stunden zwischen 1000 und 2500 mPa•s, insbesondere zwischen 1400 und 2000 mPa•s aufweisen, wobei die Viskositäten mittels eines LOW SHEAR Viskosimeters mit einem Geschwindigkeitsgradienten von 1 s$^{-1}$ gemessen werden.

6.  Heteropolysaccharid BM07 gemäß einem der vorangegangenen Ansprüche,
    gekennzeichnet dadurch, daß wäßrige Lösungen mit 0,2 Gew.-% des Heteropolysaccharids, deren pH 1,7 und deren Temperatur 25°C beträgt, eine Viskosität nach 24 Stunden zwischen 1000 und 2500 mPa•s, insbesondere zwischen 1400 und 2000 mPa•s aufweisen, wobei die Viskositäten mittels eines LOW SHEAR Viskosimeters mit einem Geschwindigkeitsgradienten von 1 s$^{-1}$ gemessen werden.

7.  Heteropolysaccharid BM07 gemäß einem der vorangegangenen Ansprüche,
    gekennzeichnet dadurch, daß wäßrige Lösungen mit 0,2% des Heteropolysaccharids, deren pH 11,8 und deren Temperatur 25°C beträgt, eine Viskosität nach 24 Stunden zwischen 1000 und 2500 mPa•s, insbesondere zwischen 1400 und 2000 mPa•s aufweisen, wobei die Viskositäten mittels eines LOW SHEAR Viskosimeters mit einem Geschwindigkeitsgradienten von 1 s$^{-1}$ gemessen werden.

8.  Heteropolysaccharid BM07 gemäß einem der vorangegangenen Ansprüche,
    gekennzeichnet dadurch, daß wäßrige Lösungen mit 0,2% des Heteropolysaccharids in destilliertem Wasser, welche während 24 Stunden einer Temperatur von 80°C unterworfen wurden, Viskositäten zwischen 500 und 2500 mPa•s, insbesondere zwischen 1000 und 2000 mPa•s aufweisen, wobei die Viskositäten mittels eines LOW SHEAR Viskosimeters mit einem Geschwindigkeitsgradienten von 1 s$^{-1}$ gemessen werden.

9.  Heteropolysaccharid BM07 gemäß einem der vorangegangenen Ansprüche,
    gekennzeichnet dadurch,
    daß es eine Molmasse zwischen 6•10$^6$ und 10•10$^6$ aufweist, vorzugsweise zwischen 6,5•10$^6$ und 9,5•10$^6$.

10. Heteropolysaccharid BM07 gemäß einem der vorangegangenen Ansprüche,
    gekennzeichnet dadurch, daß es Gruppierungen, abgeleitet von Glucose, Galactose und Brenztraubensäure, Bernsteinsaure und Essigsäure oder von Salzen dieser Säuren, enthält.

11. Heteropolysaccharid BM07 gemäß Anspruch 10,
gekennzeichnet dadurch, daß es Gruppierungen, abgeleitet von Glucose, Galactose und Brenztraubensäure, Bernsteinsäure und Essigsäure oder von Salzen dieser Säuren, entsprechend Molverhältnissen von jeweils 5-8/1-2/0,5-2/0, 5-2/0,05-2 enthält.

12. Heteropolysaccharid BM07 gemäß Anspruch 11,
gekennzeichnet dadurch, daß die Molverhältnisse jeweils 6-7,5/1-1,5/0,5-1/0,5-1/0, 05-0,2 betragen.

13. Heteropolysaccharid BM07 gemäß Anspruch 12,
gekennzeichnet dadurch, daß die Molverhältnisse 7/1/0,5-1/0,5-1/0,05-0,1 betragen.

14. Heteropolysaccharid BM07 gemäß einem der vorangegangenen Ansprüche,
gekennzeichnet dadurch, daß die Brenztraubensäure, Bernsteinsäure und Essigsäure in Form ihrer Salze vorliegen.

15. Heteropolysaccharid BM07 gemäß Anspruch 14,
gekennzeichnet dadurch, daß die Säuren in Form ihrer Natrium-, Kalium-, Calcium- oder Ammoniussalze vorliegen.

16. Verfahren zur Herstellung des Heteropolysaccharids BM07 gemäß einem der Ansprüche 1 bis 15,
gekennzeichnet dadurch, daß eine Fermentation eines Mediums, welches mindestens eine Quelle assimilierbaren Kohlenstoffs enthält, mit einem Stamm Agrobacterium tumefaciens I-736, einer seiner Rekombinanten oder einer seiner Mutanten ausgeführt wird, wobei die Quelle assimilierbaren Kohlenstoffs Glucose, Saccharose oder ein Stärkehydrolysat ist.

17. Verfahren gemäß einem der Ansprüche 15 und 16,
gekennzeichnet dadurch, daß die Konzentration der Quelle des assimilierbaren Koblenstoffs im Fermentationsmedium zwischen 1 und 100 g/l und vorzugsweise zwischen 15 und 60 g/l beträgt.

18. Verfahren gemäß einem der Ansprüche 15 bis 17,
gekennzeichnet dadurch, daß das Fermentationsmedium mindestens eine Quelle organischen Stickstoffs enthält.

19. Verfahren gemäß Anspruch 17,
gekennzeichnet dadurch, daß die Quelle organischen Stickstoffs Casein und Caseinat, Weizen-, Mais- oder Sojamehl, Hefeextrakt, die löslichen Bestandteile aus der Trockendestillation, Kartoffelproteine, Maisquellwasser und die löslichen Bestandteile des Maisquellwassers sein kann.

20. Verfahren gemäß Anspruch 19,
gekennzeichnet dadurch, daß die Quelle organischen Stickstoffs Maisquellwasser oder die löslichen Bestandteile des Maisquellwassers ist.

21. Verfahren gemäß einem der Ansprüche 18 bis 20,
gekennzeichnet dadurch, daß die Konzentration der Quelle organischen Stickstoffs im Fermentationsmedium zwischen 3 und 80 g/1 liegt.

22. Verfahren gemäß einem der Ansprüche 15 bis 21,
gekennzeichnet dadurch, daß das Fermentationsmedium mindestens ein Mineralsalz enthält.

23. Verfahren gemäß Anspruch 22,
gekennzeichnet dadurch, daß das Mineralsalz ein Sulfat oder Carbonat ist.

24. Verfahren gemäß einem der Ansprüche 21 bis 23,
gekennzeichnet dadurch, daß die Konzentration des Mineralsalzes zwischen 0,01 und 5 g/1 liegt.

**25.** Verfahren gemäß einem der Ansprüche 15 bis 24,
gekennzeichnet dadurch, daß es bei Drücken zwischen 1 und 4 bar und Temperaturen zwischen 25 und 35°C unter submersen, aeroben Bedingungen durchgeführt wird.

**26.** Verfahren gemäß einem der Ansprüche 15 bis 25,
gekennzeichnet dadurch, daß das pH des Fermentationsmediums zwischen 5 und 9, vorzugsweise zwischen 6 und 8 liegt.

**27.** Verfahren gemäß einem der Ansprüche 15 bis 26,
gekennzeichnet dadurch, daß die Ausbeuten der Fermentation 40 bis 75 Gew.-% an hergestelltem Heteropolysaccharid BM07 betragen, bezogen auf die eingesetzte Kohlenttoffquelle.

**28.** Verfahren gemäß einem der Ansprüche 15 bis 27,
gekennzeichnet dadurch, daß das Heteropolysaccharid BM07 aus der Fermentationsbrühe gemäß den folgenden Schritten abgetrennt wird:
- man erhitzt die Fermentationsbrühe auf zwischen 80 und 120°C während 10 bis 60 Minuten;
- man fällt das Heteropolysaccharid BM07 mittels einer mit Wasser mischbaren, organischen Flüssigkeit aus;
- man trennt das Heteropolysaccharid BM07 aus der organischen Flüssigkeit durch Filtration, Zentrifugation oder Flüssigkeitsentzug ab.

**29.** Verfahren gemäß Anspruch 28,
gekennzeichnet dadurch, daß die organische Flüssigkeit Ethanol, Propanol, Isopropanol, Butanol oder tert.-Butanol ist.

**30.** Verfahren gemäß einem der Ansprüche 29 und 29,
gekennzeichnet dadurch, daß das Heteropolysaccharid nach dem Abtrennen aus der Fermentationsbrühe dehydratisiert, getrocknet, zerkleinert und gesiebt wird.

**31.** Verwendung des Heteropolysaccharids BM07 gemäß einem der Ansprüche 1 bis 15 als Verdickungsmittel.

**32.** Verwendung des Heteropolysaccharids BM07 gemäß einem der Ansprüche 1 bis 15 als Suspensionsmittel.

**33.** Verwendung des Heteropolysaccharidds BM07 gemäß einem der Ansprüche 1 bis 15 in der Erdölindustrie, agrochemischen Industrie, Nahrungsmittelindustrie, Kosmetikindustrie, Papierindustrie, Textilindustrie, Sprengstoffindustrie ebenso wie in keramischen Zusammensetzungen und in Schmierstoffen, in Anstrichfarben, Klebstoffen, Tinten, Betonen, Gipsen, Haushalts- oder Industriereinigern und als Stabilisierungsmittel wäßriger Dispersionen.

**34.** Verwendung des Heteropolysaccharids BM07 gemäß einem der Ansprüche 1 bis 15 als Verdickungsmittel in Zusammensetzungen wäßriger Säuren, welche in wäßriger Lösung eine organische Säure mit einer Dissoziationskonstante pK bei 25°C größer oder gleich 2 und/oder wenigstens ein Salz einer organischen Säure oder Mineralsaure mit einem pK größer oder gleich 2 und ein Salz einer starken Base enthalten.

**35.** Verwendung des Heteropolysacchrids BM07 gemäß Anspruch 34,
gekennzeichnet dadurch, daß die Säure Ameisensäure, Essigsäure, Citronensäure oder Gerbsäure ist.

**36.** Verwendung des Heteropolysaccharids BM07 gemäß einem der Ansprüche 34 und 35,
gekennzeichnet dadurch, daß die Zusammensetzungen für die Reinigung von Oberflächen, zur Entkalkung von Porzellanoberflächen und zum Beizen von Metalloberflächen bestimmt sind.

# FIG.1

EP 0 351 303 B1

EP 0 351 303 B1

# FIG.2

viscosité
mlas

$10^4$

$10^3$

hétéropolysaccharide BM07

rhodopol 23

$10^{-1}$  1  10  $\gamma$

Gradent de vitesse